(19) Europäisches Patentamt
European Patent Office
Office européen des brevets

(11) Publication number: **0 227 351 B1**

## EUROPEAN PATENT SPECIFICATION

(45) Date of publication of patent specification: **13.11.91**

(21) Application number: **86309432.2**

(22) Date of filing: **03.12.86**

(51) Int. Cl.⁵: **G01N 33/68**, G01N 33/543,
//G01N33/547

The file contains technical information submitted
after the application was filed and not included in
this specification

(54) **Immobilized C-peptide antibody, and the preparation and use thereof.**

(30) Priority: **04.12.85 JP 273825/85**

(43) Date of publication of application:
**01.07.87 Bulletin 87/27**

(45) Publication of the grant of the patent:
**13.11.91 Bulletin 91/46**

(84) Designated Contracting States:
**BE CH DE FR IT LI SE**

(56) References cited:
**EP-A- 0 047 917**
**EP-A- 0 134 307**
**WO-A-83/02069**
**FR-A- 2 470 113**
**GB-A- 2 013 688**

**CHEMICAL ABSTRACTS, vol. 97, no. 23, 06
Dec 1982, Columbus, OH (US); W.G.REEVES
et al., p. 108, no. 193533k**

(73) Proprietor: SHIONOGI & CO., LTD.
1-8, Doshomachi 3-chome Chuo-ku
Osaka 541(JP)

(72) Inventor: **Tetsuo, Tsuji**
**200-4, Nakamachi**
**Kashihara-shi Nara(JP)**
Inventor: **Masao, Kono**
**3-25-28, Ayukawa**
**Ibaraki-shi Osaka(JP)**
Inventor: **Ken, Inouye**
**131-2-907, Arise Ikawadani-cho Nishi-ku**
**Kobe-shi Hyogo(JP)**

(74) Representative: **Allard, Susan Joyce et al**
**BOULT, WADE & TENNANT, 27 Furnival
Street
London EC4A 1PQ(GB)**

## Description

The present invention relates generally to diagnostic immunoassay. Particularly it is concerned with an immobilized antibody for use in the single-antibody immunoassay of proinsulin C-peptide in blood, urine or the like.

Immobilized antibodies are now being increasingly used in the field of immunoassay, because they are easier to handle than soluble antibodies.

Immobilization of an antibody to an insoluble solid support has hitherto been performed by utilizing physical adsorption or chemical reaction between these two depending on the purpose and conditions of the immobilization. Of the chemical methods, this one using a condensing agent represented by carbodiimide is most common.

Immobilized second antibodies have been widely used in the double antibody method. In such cases, there are few problems in immobilization of antibodies because the role of the immobilized second antibodies is limited to precipitation or separation of the antigen-antibody complex from the free antigen. Therefore the antibody is not necessarily required to be of high specificity and sensitivity.

In contrast to this, immobilized antibodies used in the single antibody method are required to be highly sensitive and specific. Because of this, it must be especially important to develop a procedure for preparing such immobilized antibodies though there are many difficulties to overcome. Therefore, if an immobilized antibody of high quality is once made available, the single antibody method should be preferred to the double antibody method, because the former is much easier to perform and requires far shorter time for assay as compared with the latter. In addition, it may be expected that the reactivity, specificity and sensitivity of antibodies could be improved by immobilization.

When an antibody is coupled with a water insoluble support by the use of such a coupling reagent as carbodiimide, the antibody may undergo intramolecular and intermolecular oligomerization to form heterogeneous products. This often accompanies denaturation of the antibody and causes deterioration of the function of the antibody Therefore, it has been thought very unlikely to expect that the properties of the antibody would be improved by immobilization.

An attempt has been made to measure C-peptide by using the single antibody method in place of the double antibody method which is currently employed, but it has not brought about any satisfactory result when immobilized antibody prepared by the carbodiimide method is used.

The conventional double antibody method usually requires a long incubation time for the first reaction and an additional time for the reaction with the second antibody. It is not easy to handle and not always sensitive enough to determine C-peptide at its critical levels. With regard to the immobilized antibody, emphasis has hitherto been placed too heavily on its easy operation and convenience in immunoassay whereas any possible improvement in the nature of antibody has never been referred to. Most of the patents (unexamined publication) and literature published thus far relate only to the methods of or the material for immobilization. None of them have dealt with immobilization an an effective means for improving the properties of the antibody itself.

GB-A-2013688 discloses an antibody immobilized via NCA (-chloroacetyl-lysine N-carboxyanhydride) on albumin-coated latex-particles.

WO-A-83/02069 discloses a method of linking an antibody via a thiol-reactive group to an amino-group of a liposome.

Therefore, the advent of a single antibody method which uses an immobilized single antibody endowed with improved reactivity and sensitivity would be most welcome.

The present invention provides an antibody immobilized on a microgranular polyacrylamide gel which is partially hydrolyzed and is coupled with a straight-chain alkylenediamine to provide free amino groups, wherein said antibody is a partially reduced form of immunoglobulin specific to human proinsulin C-peptide and having thiol groups, by the heterobifunctional cross-linking reagent m-maleimidobenzoic acid N-hydroxysuccinimide ester (MBS) which has formed two bonds, one with a thiol group of the immunoglobulin and the other with an amino group of the gel.

Another aspect of the present invention is to provide a process for preparing immobilized antibodies which comprises linking the immunoglobulin obtained from an antiserum specific to human proinsulin C-peptide by purifying the antiserum on an immunoaffinity column carrying human proinsulin C-peptide or its fragment as a ligand, followed by partial reduction to a microgranular polyacrylamide gel which is partially hydrolyzed and has been coupled with a straight-chain alkylenediamine to provide free amino groups, by the m-maleimidobenzoic acid-N-hydroxysuccinimide ester.

In developing a method of immobilization, the present inventors have found that when anti-human proinsulin C-peptide antibody is partially reduced under specific mild conditions to liberate a limited number of thiol groups and these thiol groups are cross-linked to amino groups on the surface of the aminated solid support, the immobilized antibody obtained has significantly increased reactivity and sensitivity as compared with the antibody before immobilization. They have also conducted a detailed investigation to find optimum conditions for the immobilization and to increase reproducibility of the operation as will be described in the following.

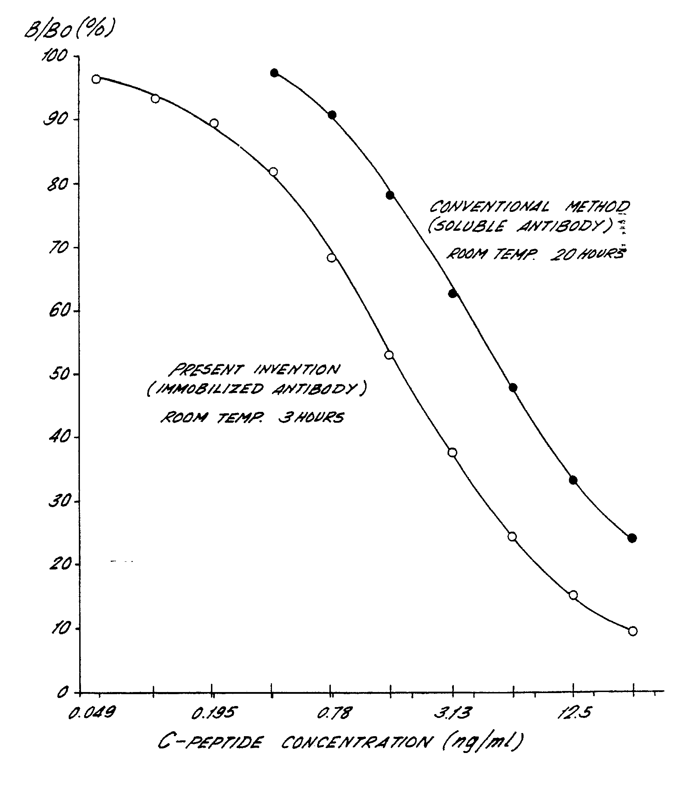

The single Figure is a graph showing a standard curve obtained with the Example of the present invention in comparison with that obtained with a comparative experiment.

As for the water-insoluble solid support, any carrier which inherently has amino groups can be used. These include aminobenzyl polyacrylamido, an aminoalkylsilane adduct of glass particles or the like.

Microgranular polyacrylamide gel having amino groups is suitably employed as the solid support here. The introduction of amino groups to the support is preferably performed by partial hydrolysis of the polyacrylamide support, followed by coupling of a straight-chain alkylenediamine to the liberated carboxyl groups of the support. Hexamethylendiamine is the straight chain alkylenediamine of choice.

Particle size of the microgranular polyacrylamide gel is important in preparing the immobilized antibody. Excessively large granules tend to sediment to the bottom of the container, while excessively small granules make separation difficult. IMMUNOBEAD Matrix (Trademark of Bio-Rad Laboratories Inc., Richmond, USA.) having particle size of 0.1μm -10μm, or preferably 1 - 5 μm is used. m-maleimidobenzoic acid N-hydroxysuccinimide ester (MBS) is the heterobifunctional cross-liking agent.

The preparation of the immobilized antibody of the present invention starts preferably with coupling of hexamethylendiamine to the carboxyl groups of the partially hydrolyzed microgranular polyacrylamide gel with 1-ethyl-3-(3-dimethylaminopropyl)carbodiimide (EDC) to introduce amino groups to the support. To these amino groups is coupled the heterobifunctional cross-linking reagent to activate the solid support for the subsequent reaction with thiol groups.

The antibody to be immobilized, the partially reduced form of immunoglobulin specific to human proinsulin C-peptide, may be prepared by first purifying an anti-human proinsulin C-peptide antiserum on an immunoaffinity column with human proinsulin C-peptide or its fragment as ligand, followed by partial reduction with mercaptoethylamine to liberate a limited number of thiol groups. Rabbit immunoglobulin G is preferred because it has a single disulfide bond susceptible to mild reduction. This disulfide bond might be the one linking the two H-chains.

The last step of the immobilization is the reaction between the reduced antibody and the activated solid support to produce the desired immobilized antibody.

The standard curve obtained with the immobilized antibody is compared with that of the conventional double antibody method using the original antiserum and demonstrates a significant increase in the sensitivity of the assay over the conventional method.

In the following paragraphs the present inventions will be explained in more detail by way of an Example and comparison with the conventional procedure.

Example

(1) Purification of Antibody

Rabbit antiserum (0.5 ml) specific to human proinsulin C-peptide was charged onto a column (volume: 1.8 ml) packed with an immunoadsorbent [Sepharose 4B (Registered Trademark) being coupled to human proinsulin C-peptide or its fragment (for instance, C-peptide (7-24))] and the column was washed with fifteen volumes of 50 mM Tris-HC1 buffer (pH 7.5).

Then the specifically, bound immunoglobulin was eluted with 6mM HC1 to give the purified antibody (IgG).

(2) Reduction of Antibody

Five hundred and forty (540) μ1 of 0.1 M potassium phosphate buffer (pH 6.0) containing 0.5 mg of the purified antibody was combined with 60 μ1 of 40 mM mercaptoethylamine and the mixture was allowed to react at 37°C for 60 minutes. Excess mercaptoethylamine was removed from the reaction mixture by gel filtration on a column of Sephadex G-25 medium (Registered Trademark) to give the reduced antibody.

The reduced antibody thus obtained was found to have two thiol groups per molecule of the IgG on the average.

(3) Amination of Water Insoluble Solid Support

A mixture of 200 mg of Immunobead (Registered Trademark) matrix having particle size distribution of 1-7.5 μm and 2.25 mole of hexamethylenediamine in 20 ml of 3 mM phosphate buffer (pH 6.3) was stirred at 4°C for one hour.

To this was added 40 mg of EDC [1-ethyl-3(3-dimethylaminopropyl) carbodiimide] hydrochloride and the mixture was maintained at pH 5.0 for about 30 minutes by the addition of 1 N HCl thereto and then allowed to react at 4°C for two hours. This procedure was repeated until the carboxyl groups on the solid support were completely lost.

## (4) Preparation of Maleimidobenzoylated (MB) Support

Twenty (20) mg of the aminated insoluble solid support was allowed to react with 10 $\mu$ mole of MBS (m-maleimidebenzoic acid N-hydroxysuccinimide ester, about five-fold molar excess based on the amino groups of the solid support) in 0.1 M phosphate buffer (pH 7.0; final volume 1.6 ml) at 30°C for one hour.

Removal of the unreacted MBS by washing three times with a mixture of 0.1 M sodium phosphate buffer (pH 6.0) and tetrahydrofran (THF; 1:1) containing 5 mM EDTA and further three times with 0.1 M sodium phosphate buffer (pH 6) containing 5 mM EDTA gave a suspension of MB Support of a final volume of 2.25 ml.

## (5) Immobilization of Antibody

To the above-mentioned reduced antibody whose amount corresponds to O.D. (adsorption at 280 nm x ml) = 0.1 was added 0.5 ml of the above-mentioned suspension of MB support. The final volume was adjusted to 1 ml with 0.1 M sodium phosphate buffer (pH 6.0) containing 5 mM EDTA and the resulting mixture was allowed to react at 4°C for twenty hours. MB groups which had remained unreacted on the water insoluble solid support were deactivated by the treatment at 30°C for 20 minutes with 2-mercaptoethanol three times the amount of the unreacted groups.

The immobilized antibody was washed five times with 50 mM phosphate/NaCl buffer (pH 7.5) and then five times with 0.01 M phosphate buffer (pH 7.5) containing 0.9 % NaCl, 0.5 % BSA, 0.01 M EDTA and 0.05 % sodium azide (hereinafter, the latter buffer is called simply as "the Buffer". This is equivalent to a constituent of the kit "C-peptide test Shionogi" available from Shionogi & Co. Ltd.) and stored at 4°C after being adjusted to an appropriate volume by the addition of the Buffer.

## (6) Measurement of the potency and sensitivity

The following solutions and suspension are prepared by utilizing the reagents which constitute the above-mentioned "C-peptide test Shionogi".

a. Standard human C-peptide solutions (0.2 - 50 ng/ml).

b. Iodotyrosylated [125I] human C-peptide solution.

c. Immobilized antibody, a suspension obtained in the above step(5).

One tenth ml of each of the a, b and c were mixed together and stirred vigorously to start the reaction. The mixture was allowed to stand at room temperature for 3 hours. For B/F separation, the mixture was centrifuged (2,000 g x 7 min.) and the supernatant was removed by aspiration. The radioactivity of the insoluble residue was measured by the conventional method. The measurements performed at varying C-peptide concentrations permitted the estimation of the potency and of the immobilized antibody.

## Comparative Experiment (Conventional assay method)

One tenth (0.1) ml of the rabbit antiserum, which was specific to human proinsulin C-peptide and used as the starting material in Step (1) of Example, was mixed with a solution containing 0.1 ml each of the above a and b ,and 0.5 ml of the Buffer. The mixture was allowed to stand at 20°C for 20 hours (the first reaction).

To this mixture was added 0.1 ml of a suspension of anti-rabbit immunoglobulin antibody-bound gel. The mixture was kept standing thereafter at 20°C for one hour (the second reaction) and then centrifuged (1, 500 g x 5 min.). The subsequent procedure is similar to that described in Step(6) of Example.

A comparison was made between the standard curves obtained by the two operations (according to the present invention and the conventional method). The result is shown in the attached Figure.

## Effect of Invention

As described above, the present invention has made it possible to measure proinsulin C-peptide by the single antibody method. According to the present invention, the time required for the measurement is 7 - 8 times shorter and the sensitivity of the measurement is 2 - 4 times higher as compared with the conventional double-antibody method.

Thus, it can be seen that purification of an antiserum specific to human proinsulin C-peptide by means of immunoaffinity chromatography on a column carrying human proinsulin C-peptide or a fragment thereof as a ligand and the subsequent partial reduction and immobilization to a water-insoluble solid support with the aid of a heterobifunctional cross-linking reagent afford an excellent immobilized antibody which shows higher specificity and sensitivity than the original antiserum. A radioimmunoassay kit for the measurement of human proinsulin C-peptide in body fluids may comprise said immobilized antibody, standard human proinsulin C-peptide solution, a radioactively labelled derivative of human proinsulin C-peptide and a buffer.

## Claims

1. An antibody immobilized on a microgranular polyacrylamide gel which is partially hydrolyzed and is coupled with a straight-chain alkylene diamine to provide free amino groups, wherein said antibody is a partially reduced form of immunoglobulin specific to human proinsulin C-peptide and having thiol groups, by the heterobifunctional cross-linking reagent m-maleimidobenzoic acid N-hydroxysuccinimide ester (MBS) which has formed two bonds, one with a thiol group of the immunoglobulin and the other with an amino group of the gel.

2. A process for preparing an immobilized antibody as claimed in claim 1 which comprises linking the immunoglobulin obtained from an antiserum specific to human proinsulin C-peptide by purifying the antiserum on an immunoaffinity column carrying human proinsulin C-peptide or its fragment as a ligand, followed by partial reduction, to a microgranular polyacrylamide gel which is partially hydrolyzed and has been coupled with a straight-chain alkylene diamine to provide free amino groups, by the heterobifunctional cross-linking reagent m-maleimidobenzoic acid N-hydroxysuccinimide ester (MBS).

3. An immunoassay for human proinsulin C-peptide based on the single antibody method which comprises the use of an immobilized antibody as claimed in claim 1.

4. A radioimmunoassay kit for assaying human proinsulin C-peptide in body fluids which comprises an immobilized antibody as claimed in claim 1.

5. The use of an antibody as claimed in claim 1 in an immunoassay for human proinsulin C-peptide.

## Revendications

1. Un anticorps immobilisé sur un gel microgranulaire de polyacrylamide qui est partiellement hydrolysé et couplé à une alkylediamine à chaine linéaire pour fournir des groupement amino libres, dans lequel ledit anticorps est une forme partiellement réduite d'immunoglobuline spécifique du C-peptide de la proinsuline humaine ayant des groupements thiols, par l'agent de couplage hétérobifonctionnel N-hydroxysuccinimide ester de l'acide 3-maléimidobenzoique (MBS) qui a formé deux liaisons, une avec un groupement thiol de l'immunoglobuline et l'autre avec un groupement amine du gel.

2. Un procédé pour préparer un anticorps immobilisé selon la revendication 1 qui comprend le greffage d'une immunoglobuline obtenue à partir d'un antisérum spécifique du C-peptide de la proinsuline humaine par purification de l'antisérum sur une colonne d'immunoaffinité portant le C-peptide de proinsuline humaine ou ses fragments comme ligand, suivie par une réduction partielle, sur un gel de polyacrylamide microgranulaire qui est partiellement hydrolysé et a été couplé par le réactif de couplage hétérobifonctionnel qui est le N-hydroxysuccinimide ester de l'acide 3-maléimidobenzoique à une diamine à chaine linéaire pour fournir des groupements amino libres.

3. Un dosage immunologique du C-peptide de proinsuline humaine basé sur la méthode du simple anticorps qui comprend l'utilisation d'un anticorps immobilisé selon la revendication 1.

4. Un dosage immunologique par radioactivité pour mesurer le C-peptide de la proinsuline humaine dans les fluides corporels et qui comprend un anticorps immobilisé selon la revendication 1.

5. L'utilisation d'un anticorps selon la revendication 1 dans un test immunologique pour le C-peptide de la proinsuline humaine.

## Patentansprüche

1. Antikörper, der auf einem mikrokörnigen Polyacrylamidgel, welches teilweise hydrolysiert und mit einem geradkettigen Alkylendiamin gekuppelt ist, um freie Aminogruppen zur Verfügung zu stellen, wobei der Antikörper eine partiell reduzierte Form von Immunoglobulin ist, das spezifisch für humanes Proinsulin C-Peptid ist und Thiolgruppen aufweist, durch das heterobifunktionelle Vernetzungsreagens m-Maleinimidobenzoesäure-N-hydroxysuccinimidester (MBS) immobilisiert ist, der zwei Bindungen gebildet hat, eine mit einer Thiolgruppe des Immunoglobulins und die andere mit einer Aminogruppe des Gels.

2. Verfahren zur Herstellung eines immobilisierten Antikörpers nach Anspruch 1, gekennzeichnet durch Verbinden des Immunoglobulins, welches aus einem Antiserum, das für humanes Proinsulin C-Peptid spezifisch ist, durch Reinigung des Antiserums auf einer Immunoaffinitätssäule, die humanes Proinsulin C-Peptid oder dessen Fragment als einen Liganden trägt, mit anschließender partieller Reduktion, erhalten ist, an ein mikrokörniges Polyacrylamidgel, welches teilweise hydrolysiert ist und mit einem geradkettigen Alkylendiamin gekuppelt worden ist, um freie Aminogruppen zur Verfügung zu stellen, wobei die Verbindung mit Hilfe des heterobifunktionellen Vernetzungsreagens m-Maleinimidobenzoesäure-N-hydroxysuccinimidester (MBS) erfolgt.

3. Immununtersuchung für humanes Proinsulin C-Petid, basierend auf dem einzigen Antikörperverfahren, welches die Verwendung eines immobilisierten Antikörpers nach Anspruch 1 umfaßt.

4. Radioimmununtersuchungspackung zur Untersuchung von humanem Proinsulin C-Peptid in Körperfluiden, welches einen immobilisierten Antikörper nach Anspruch 1 enthält.

5. Verwendung eines Antikörpers nach Anspruch 1 in einer Immununtersuchung für humanes Proinsulin C-Peptid.

B/Bo (%)

CONVENTIONAL METHOD
(SOLUBLE ANTIBODY)
ROOM TEMP. 20 HOURS

PRESENT INVENTION
(IMMOBILIZED ANTIBODY)
ROOM TEMP. 3 HOURS

C-PEPTIDE CONCENTRATION (ng/ml)